# EUROPEAN PATENT APPLICATION

(11) **EP 4 289 421 A1**
(43) Date of publication of application: **13.12.2023**
(21) Application number: 23177509.9
(22) Date of filing: 06.06.2023
(51) Int. Cl.: A61K 31/164, A61K 31/4045, A61P 29/00

(54) **COMBINATION OF PALMITOYLETHANOLAMIDE (PEA) AND MELATONIN FOR THE TREATMENT OF MAST CELL HYPER-REACTIVITY**

(30) Priority: 07.06.2022 IT 202200011957
(71) Applicant: Erbozeta S.p.A., 47894 Chiesanuova (SM)
(72) Inventor: AURIEMMA, Ferdinando, I-47826 VERUCCHIO (Rimini) (IT); ZAVAGLIA, Roberto, 47891 FALCIANO (SM)
(74) Representative: Rambelli, Paolo

(57) **Abstract**

Synergistic composition comprising PEA (palmitoylethanolamide) and melatonin for use in the treatment of pathologies sustained by, or attributable to, mast cell hyper-reactivity and in particular in the treatment or as an adjuvant in the treatment of pain and/or inflammation.

## Description

### FIELD OF THE INVENTION

The present invention relates to a composition comprising the combination of PEA (palmitoylethanolamide) and melatonin for use in the treatment of pathologies sustained by, or attributable to, mast cell hyper-reactivity, in particular for use in the treatment of pain and inflammation of various kinds, especially if chronic.

Given the presence of melatonin, traditionally supplemented in the evening, the composition object of the present invention is peculiar because it is intended for use at any time of the day.

The aforementioned use may be implemented through formulations for food supplements, foods for special medical purposes, drugs or medical devices.

### PRIOR ART

Mast cells are immune cells that activate in response to stimuli of various kinds, releasing by degranulation a wide range of pro-pain and pro-inflammatory mediators, including histamine, TNF-α and IL-6: being located near the nerves and blood vessels, they are heavily involved in the development and maintenance of pain and inflammation.

Mast cells are considered crucial in the chronicity of painful and inflammatory states: their excessive degranulation, on the one hand, sensitizes the nociceptive neurons, which undergo a lowering of the activation threshold and begin to respond more easily to endogenous and exogenous stimuli, on the other hand, it induces nociceptive neurons and other surrounding cells, such as macrophages or microglia cells, to release biochemical mediators that eventually sensitize the mast cells themselves.

A vicious masto-neural circuit is formed, also involving the vascular system, which may have effects inside and outside the nervous system as well as in both the peripheral and central nervous system, supporting mast cell activation, and therefore pain and inflammatory processes, over time. The masto-neural interaction is even more relevant and problematic in the case of neurological damage, since this leads to a greater presence, by infiltration, of mast cells in the nervous system.

This pathophysiological scenario has given rise to a real line of research on mast cell inhibitors, intended as the last frontier in the treatment of chronic pain and inflammation as they are aimed at a basic mechanism - the hyperactivation of mast cells - which brings together painful and inflammatory pathologies also very different from each other. They represent a versatile tool which, in monotherapy or adjuvant therapy, may increase the possibilities of achieving satisfactory symptom control without having to resort to more aggressive pharmacological therapies, which are often very effective but also poorly tolerable.

PEA is one of the most studied mast cell inhibitors. It is a physiological substance chemically similar to an endocannabinoid but functionally considered an indirect cannabinergic. It is produced by different types of cells, in particular those of the nervous and immune systems, to raise the pain threshold, limit the severity and favor the resolution of inflammatory processes. It performs a multi-target action, not only on mast cells, with at least two main mechanisms: direct activation of the PPAR transcription factor and indirect activation of the CB1 and CB2 cannabinoid receptors.

Melatonin is another physiological substance whose influence on mast cells is being studied. It is produced and secreted by the pineal gland as a hormone, but may also be released locally by immune cells such as mast cells. In addition to regulating the sleep-wake cycle, which is its primary activity, it plays an important role in reducing oxidative stress and modulating pain and inflammatory processes: it neutralizes free radicals both directly and by increasing antioxidant enzymes; it mainly activates specific melatonin receptors (MT1 and MT2) but its positive interaction with GABA and opioid receptors, as well as a negative interaction with the NMDA receptor, is also known.

Particularly relevant is the fact that melatonin may be produced, stored and released by mast cells, at least those of murine origin, as a sensor of their degree of local activation: when the concentration of extracellular melatonin exceeds a certain threshold, which is a sign of excessive degranulation, the melatonin receptors on the surface of the mast cell would be adequately activated to reduce the degranulation itself.

Taken together, PEA and melatonin have a basal production that follows different rhythms: the first tends to be synthesized more during the day than at night, increasing within the limits of availability during painful and inflammatory states, the second is instead synthesized much more at night than during the day. Relevant is the fact that both tend to be deficient in the course of chronic pain and inflammatory diseases, thus leading to a loss of control over mast cell activation.

PEA and melatonin are therefore physiological substances which, individually, may or should be supplemented as part of a pain-relieving and anti-inflammatory therapy for better symptom control, and which would share the ability to inhibit mast cell activity. Before the present invention, their combined use had never been considered and it was therefore unknown whether their complex mechanisms of action synergized to determine a greater therapeutic effect and, in particular, a greater inhibition of the variegated mast cell degranulation in humans.

### SUMMARY OF THE INVENTION

The object of the invention is a synergistic composition useful for the treatment or as an adjuvant in the treatment of mast cell hyper-reactivity which is the cause or concomitant cause of various pathologies, in particular chronic painful and inflammatory ones. The features of the composition are defined in the following claims, which form part of the present description.

The present invention is based on the discovery that PEA and melatonin, in a wide range of weight ratios, exert a strongly synergistic inhibition against mast cells, immune cells whose activation is decisive for triggering and sustaining pain and inflammatory processes.

An in vitro study, purposely conducted on a line of human mast cells, revealed that the combination of PEA and melatonin, compared to these substances individually, more markedly reduces the release of important pro-pain and pro-inflammatory mediators by the activated mast cells. The effects of the combination were extraordinarily greater than the sum of the effects of the individual substances, in a wide range of weight ratios.

Considering that PEA tends to be produced more during the day while melatonin is produced much more at night, that both tend to be deficient in chronic pain and inflammatory states, and that melatonin is traditionally supplemented in the evening, the use of the combination at any time of the day is a peculiar feature of the present invention as it brings mast cell control to a higher degree which may not be reproduced physiologically: the combination, in fact, raises the levels of PEA and melatonin *at the same time* so as to compensate for both the imbalance and the shortage thereof, and therefore favor the synergism thereof.

The composition of the present invention is particularly intended for the treatment of painful and inflammatory states of various types, in particular those with a chronic course given the crucial role of mast cell hyper-reactivity precisely in the chronicization of pain and inflammation.

The composition determines a high mast cell control to which mast cell-independent effects are also plausibly associated, resulting on the one hand in the gradual shutdown of the vicious masto-neural circuit that sustains pain and inflammation over time, on the other in a multi-target treatment of the symptoms: it allows therefore, a causal and not simply symptomatic treatment of pathologies sustained by, or attributable to, mast cell hyper-reactivity.

The use of the composition object of the present invention, also as a therapeutic adjuvant, increases the chances of achieving satisfactory control of the painful and inflammatory symptoms without having to resort to more aggressive pharmacological therapies, which are often very effective but also poorly tolerable.

### SUMMARY DESCRIPTION OF THE DRAWINGS

The accompanying drawings show the results of the in vitro study, described in greater detail below.

In particular:
- Fig. 1 illustrates the histograms of mast cell degranulation in response to stimulation with compound 48/80;
- Fig. 2 illustrates the histograms of mast cell degranulation in response to stimulation with substance P.

In the histograms:
MC = control mast cells
MCA = activated mast cells
MEL = melatonin
PEA = palmitoylethanolamide

### EXPERIMENTAL ANALYSIS

A human mast cell line (HMC-1.1) was activated both by compound 48/80 (a potent synthetic stimulant, available from Sigma-Aldrich) and by substance P (potent physiological stimulant, CAS number 33507-63-0). The release of pro-pain and pro-inflammatory mediators, such as histamine, TNF-α and IL-6, was then measured in the absence of PEA and melatonin, in the presence of PEA or melatonin alone as well as in the presence of PEA + melatonin.

The use of mast cells of human origin, compared to classic murine mast cells, has allowed to measure, with high reliability, the release not only of histamine but also of protein mediators such as TNF-α and IL-6. The in vitro analysis then made it possible to evaluate the effects of the combination at the mast cell level in a highly specific and precise manner. A direct study on a biological organism, ultimately humans, would have been insensitive as the variation of pro-pain and pro-inflammatory mediators, detected in the blood or in any body fluid, would have depended on an action of PEA and melatonin on various cell types, and not exclusively on mast cells.

As expected, both compound 48/80 and substance P induced a major release of all mediators tested. Quite unexpected was instead the fact that PEA and melatonin, added individually, determined a comparable reduction in the release of mast cell mediators (this especially supports the role of melatonin, less studied than PEA, in mast cell control). Finally, totally unexpected is the discovery of the fact that the PEA + melatonin combination determines an extraordinarily stronger inhibition of mast cells not only with respect to the same substances individually but also, and above all, with respect to the theoretical sum of their respective effects.

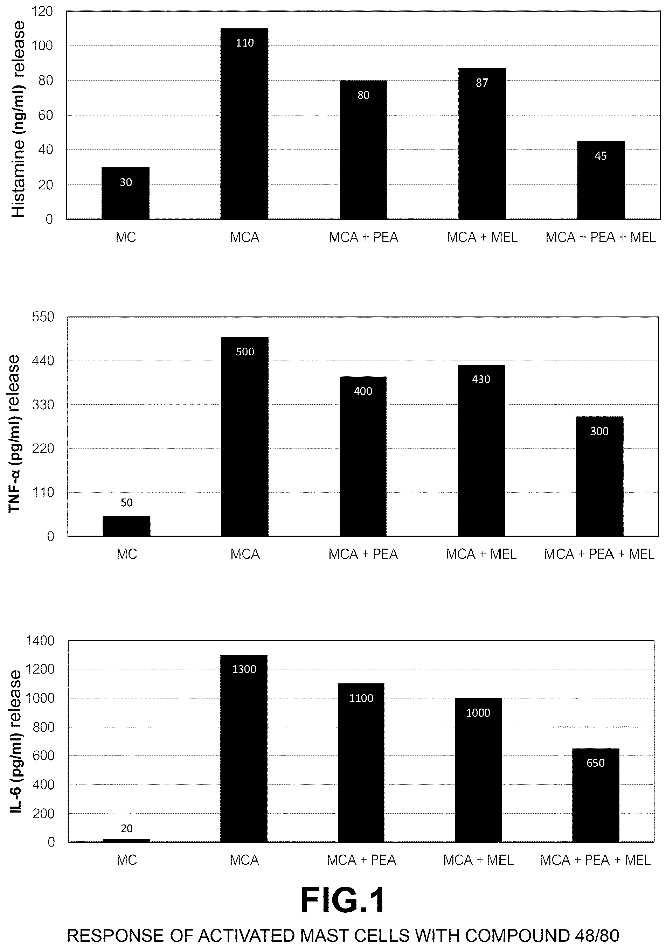

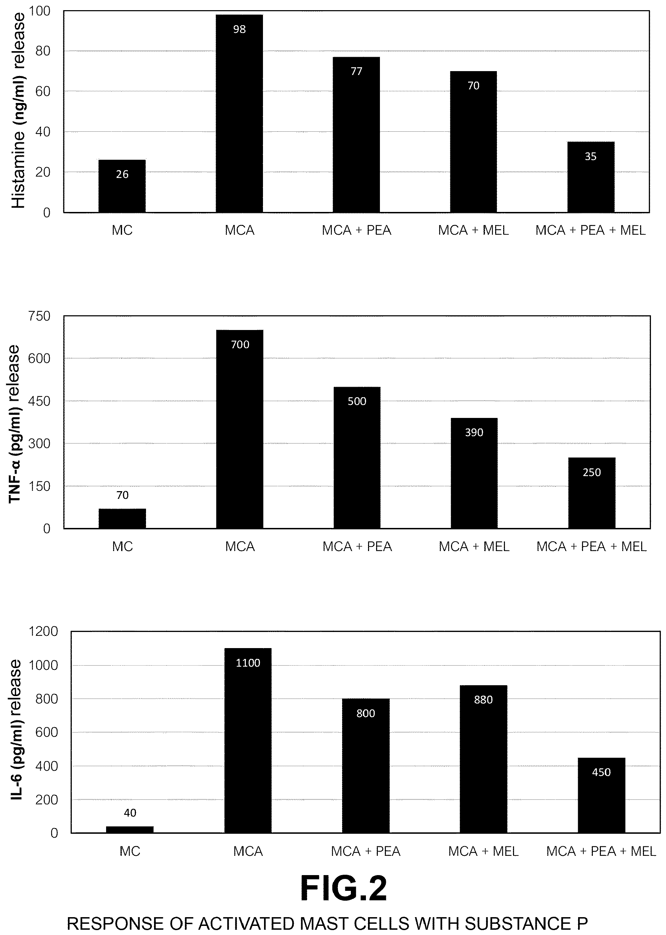

These results were observed in the presence of compound 48/80, confirmed in the presence of substance P and obtained with PEA + melatonin at different weight ratios. Figs. 1 and 2 show that, using PEA + melatonin in a micromolar ratio of 6000: 1, an average gain of mast cell inhibition equal to 22% was obtained compared to the simple sum of the effects of PEA and melatonin individually, demonstrating a remarkable synergy. This was the best result within the weight ratios tested.

In particular, the graphs of Figs. 1 and 2 indicate a significant difference for MCA + PEA + MEL compared to all other groups as well as compared to the sum of MCA + PEA and MCA + MEL (p < 0.05), as well as for MCA + PEA and MCA + MEL compared to MCA and MC (p < 0.05). Non-significant difference between MCA + PEA and MCA + MEL.

### DESCRIPTION OF THE INVENTION

The present invention is based on the discovery that PEA and melatonin counteract, with different but strongly synergistic mechanisms, the activation of mast cells which triggers and supports pain and inflammatory processes. Therefore, the invention relates to a composition comprising as active ingredients (or consisting of) the combination of PEA and melatonin, for use in the treatment of pathologies sustained by, or attributable to, mast cell hyper-reactivity, such as those involving chronic pain and inflammation.

An in vitro study revealed that the combination between PEA and melatonin determines a mast cell inhibition, evaluated in terms of mediators released in response to a strong stimulation, extraordinarily greater than the sum of the mast cell inhibitions produced by PEA and melatonin individually, which is a sign of a strong synergism between the complex mechanisms of action of these substances.

The combination allows for greater mast cell control in terms not only of strength but also of timing of the inhibition. PEA acts mainly by direct activation of the transcription factor PPAR, melatonin mainly activates the melatonin receptors on the plasma membrane of the mast cell: a slower but prolonged gene modulation effect, associated with an effect that is also cytoplasmic, certainly more direct, may in fact determine a mast cell inhibition on the one hand stronger, on the other hand sustained and rapid at the same time.

The composition of the present invention is intended for use at any time of the day (morning, afternoon or evening), a particularly relevant aspect considering that melatonin supplementation as a pain reliever and anti-inflammatory is described in the literature always before a state of non-vigilance such as sleep or surgical anesthesia. In line with its traditional use in helping to fall asleep, dosages of melatonin are generally in the range of 1-10 mg and such as to cause loss of alertness.

However, melatonin is continuously present and circulating in the body: its plasma levels, dependent on the productive activity of the pineal gland, are very low in the morning while they rise in the evening until they reach a peak during the night, promoting sleep. It is believed that endogenous melatonin, even at low daytime levels, helps to control pain and inflammation, and therefore its deficiency represents a pro-pain and pro-inflammatory factor for the body.

Based on the kinetic profiles of exogenous melatonin in humans, it is therefore possible to establish routes of administration and dosages which are compatible with the levels of endogenous hormone (for example 0.05 mg orally) in order to improve its action against pain and inflammation even during the day and without causing drowsiness: when choosing for daytime use (morning or afternoon), it should be taken into consideration that one is very sensitive to exogenous melatonin (0.1 mg orally is sufficient to significantly raise normal plasmatic levels which are in the order of a few ng/ml); on the other hand, it should also be considered that the minimum dosage of melatonin capable of promoting falling asleep is currently considered to be 1 mg orally before going to bed (EU regulation 432/2012).

Considering that PEA tends to be produced more during the day while melatonin is produced more at night and both tend to be deficient in chronic pain and inflammatory states, the use of the combination at any time of the day brings mast cell control to a higher degree which may not be reproduced physiologically: the combination, in fact, raises the levels of PEA and melatonin at *the same time* so as to compensate for both the imbalance and the shortage thereof, and therefore favor the synergism thereof.

The wide choice on the time of use is particularly relevant in the morning or afternoon, when a greater mast cell excitability is observed also because the inhibitory contribution of melatonin is naturally and strongly decreased. Evening use, however, may be further beneficial by virtue of the fact that melatonin, integrating the physiological one in this circumstance, promotes and improves sleep: it is in fact known that improving the quality of sleep helps reduce pain and inflammation perceived.

Since PEA and melatonin perform a multi-target action at both molecular and cellular level, it is then plausible that their combination determines a reduction of pain and inflammation not only through a strong mast cell inhibition, but also through an influence on pathophysiological mechanisms that do not directly involve the mast cells, resulting on the one hand in the gradual shutdown of the vicious masto-neural circuit which helps to sustain pain and inflammation over time, on the other hand in a more effective symptomatic treatment because it is multi-target.

The composition of the present invention is therefore intended for the treatment of pathologies for which mast cell hyper-reactivity is well documented as a concurrent or triggering cause. This composition is therefore intended for the causal and not simply symptomatic treatment of pathologies such as: peripheral neuropathies, migraine, fibromyalgia, osteoarthritis, cancer pain, allergic diseases, psoriasis, various types of chronic pelvic pain including Crohn's disease, irritable bowel syndrome, endometriosis, chronic prostatitis and vulvodynia, and by definition mast cell activation syndrome and mastocytosis.

Considering that PEA and melatonin may be administered in different formulations, with the aim above all of improving the bioavailability thereof, the present invention also includes PEA and melatonin which have features, or are associated with particular excipients, so as to improve the kinetic profile of the single active ingredients or of their combination.

PEA may be, by way of a non-limiting example, micronized or ultramicronized as well as combined with excipients which, individually or combined, promote or regulate the bioavailability thereof, such as phospholipids, polyglycerol esters of fatty acids, mono- and diglycerides of fatty acids, medium chain triglycerides, fractionated coconut oil, polyethoxylated castor oil, lime oil, olive oil, oat oil, sunflower lecithin, vitamin E acetate, silicon dioxide, microcrystalline cellulose, croscarmellose sodium, polyvinylpyrrolidone, magnesium stearate, sorbitol powder, sucrose palmitate, polysorbate 80, colloidal anhydrous silica.

Melatonin may be, by way of a non-limiting example, micronized or ultramicronized as well as combined with excipients which, individually or combined, favor or regulate the bioavailability thereof, such as sucrose, corn starch, hydroxypropyl cellulose, talc, shellac.

The combination between PEA and melatonin is intended for reproduction in any form of administration, with particular attention to formulations for oral, sublingual, rectal, transdermal and topical use, relating to food supplements, foods for special medical purposes, drugs or medical devices.

Considering that PEA is clinically used in the order of milligrams and that melatonin is clinically usable in the order of micrograms, and taking into account that the effects of combined supplementation depend on many variables (endogenous levels depending on the pathology and the time of intake, form of administration of the individual substances as well as route of administration of the product), the present invention includes, per single dose, an amount of PEA within the range of 50-2400 mg, preferably from 200 to 1200 mg, combined with an amount of melatonin within in the range of 0.01-10 mg, preferably from 0.1 to 5 mg, with quantitative ratios (PEA/melatonin, mg/mg) falling within the range of 100:1 - 120000:1 for oral and sublingual use, 100:1 - 10000:1 for rectal, transdermal and topical use.

In oral and sublingual use, the quantitative ratio between PEA and melatonin is preferably from 1000:1 to 50000:1, more preferably from 2000:1 to 15000:1 and even more preferably from 4000: 1 to 8000:1, with the latter representing the range in which synergism is maximized. In rectal, transdermal and topical administration, the weight ratio of PEA to melatonin is preferably from 1000:1 to 8000:1, more preferably from 4000:1 to 8000:1.

The dosages of the individual substances must in any case be established in such a way as to guarantee not only the efficacy but also the tolerability of the product (melatonin, for example, may cause drowsiness if administered at a dosage of ≥ 0.5 mg orally during the day).

Examples of possible applications of the present invention are: tablets for oral use with 600 mg micronized PEA and 0.1 mg melatonin or sticks with powder for sublingual use with 300 mg ultra-micronized PEA and 0.05 mg melatonin, to be taken twice daily in the morning and evening; sachets with powder for oral use with hydrodispersible PEA 1200 mg and melatonin 0.2 mg, to be dissolved in water and taken once a day in the evening; enemas with water-dispersible PEA 1000 mg and melatonin 0.4 mg or transdermal patches with PEA 1500 mg and prolonged-release melatonin 3 mg, to be used at least once a day; pre-filled syringes for joint infiltration with hydrodispersible PEA 200 mg and melatonin 0.05 mg to be used once a week in cycles of three consecutive weeks.

Ultimately, the present invention allows optimal control of mast cell activity and of painful and inflammatory symptoms, particularly in chronic patients. The tolerability profile is also excellent as PEA and melatonin, appropriately dosed according to the chosen route and time of administration, have no particular side effects.

The ultimate benefit of the present invention, by virtue of all the aforesaid features, therefore consists in increasing the possibilities of achieving satisfactory control of the painful and inflammatory symptoms without having to resort to more aggressive pharmacological therapies, which are often very effective but also less tolerable.

By way of non-limiting example, the invention may be implemented by means of the following dosage forms:

**Example 1, tablet for oral use comprising:**
- micronized PEA, 600 mg
- melatonin, 0.1 mg
- microcrystalline cellulose, 180 mg
- croscarmellose sodium, 130 mg
- polyvinylpyrrolidone, 30 mg
- magnesium stearate, 8 mg

**Example 2, sachet with powder for oral use comprising:**
- PEA, 1200 mg
- melatonin, 0.2 mg
- polyethoxylated castor oil, 65 mg
- sunflower lecithin, 0.4 mg
- medium chain triglycerides, 0.4 mg
- lime oil, 0.4 mg
- olive oil, 0.4 mg
- oat oil, 0.4 mg
- vitamin E acetate, 0.4 mg
- colloidal anhydrous silica, 12 mg

**Example 3, stick with powder for sublingual use comprising:**
- ultramicronized PEA, 300 mg
- melatonin, 0.05 mg
- sorbitol powder, 250 mg
- sucrose palmitate, 10 mg
- polysorbate 80, 3 mg

In summary, the salient aspects of the invention may be summarized as follows:
a. Composition comprising palmitoylethanolamide (PEA) and melatonin for use in the treatment or as an adjuvant in the treatment of pain and/or inflammation in a human subject.
b. Composition for use according to item a), in formulation for oral, sublingual, rectal, transdermal or topical use comprising, per single dose, from 50 to 2400 mg of PEA, preferably from 200 to 1200 mg, and from 0.01 to 10 mg of melatonin, preferably from 0.1 to 5 mg.
c. Composition for use according to item a) or b), in formulation for oral or sublingual use comprising PEA and melatonin in a weight ratio of PEA/melatonin from 100:1 to 120000:1, preferably from 1000:1 to 50000:1, more preferably from 2000:1 to 15000:1 and even more preferably from 4000:1 to 8000:1, the latter representing the range in which synergism is maximized.
d. Composition for use according to item a) or b) in formulation for sublingual use, comprising PEA and melatonin in a weight ratio of PEA/melatonin from 100:1 to 25000:1, preferably from 2000:1 to 10000:1, more preferably from 4000:1 to 8000:1.
e. Composition for use according to item a) or b) in rectal, transdermal or topical administration, comprising PEA and melatonin in a weight ratio of PEA/melatonin from 100:1 to 10000:1, preferably from 1000:1 to 8000:1, more preferably from 4000:1 to 8000:1.
f. Composition for use according to any one of items a) to e) in the treatment of pain and inflammation of various kind, in particular in cases of post-operative pain, neuropathies such as radiculopathy, carpal tunnel syndrome, diabetic neuropathy and post-herpetic neuralgia, migraine, fibromyalgia, osteoarthritis, cancer pain, allergic inflammation and various types of chronic pelvic pain such as Crohn's disease, irritable bowel syndrome, endometriosis and chronic prostatitis.
g. Composition for use according to any one of items a) to f), in administration at any time of the day (morning, afternoon or evening).
h. Composition for use according to any one of items a) to g), further comprising a pharmaceutically or nutraceutically acceptable or food vehicle or excipient.
i. Composition for use according to any one of items a) to h), further comprising excipients selected from phospholipids, polyglycerol esters of fatty acids, mono- and diglycerides of fatty acids, medium-chain triglycerides, fractionated coconut oil, polyethoxylated castor oil, lime oil, olive oil, oat oil, sunflower lecithin, vitamin E acetate, silicon dioxide, microcrystalline cellulose, croscarmellose sodium, polyvinylpyrrolidone, magnesium stearate, sorbitol powder, sucrose, sucrose palmitate, polysorbate 80, anhydrous colloidal silica, corn starch, hydroxypropyl cellulose, talc, shellac, and mixtures thereof.
j. Composition for use according to any one of items a) to i), in which the PEA is micronized with a particle size of less than 10 µm or ultramicronized with a particle size of less than 5 µm.
k. Composition for use according to any one of items a) to j), in which melatonin is micronized with a particle size of less than 10 µm or ultramicronized with a particle size of less than 5 µm.

### BIBLIOGRAPHICAL REFERENCES

Gupta K, Harvima IT. Mast cell-neural interactions contribute to pain and itch. Immunol Rev. 2018 Mar;282(1):168-187
Skaper SD et al. Mast cells, glia and neuroinflammation: partners in crime? Immunology. 2014 Mar;141(3):314-27
Xu Y, Chen G. Mast cell and autoimmune diseases. Mediators Inflamm. 2015;2015:246126
De Filippis D et al. New insights in mast cell modulation by palmitoylethanolamide. CNS Neurol Disord Drug Targets. 2013 Feb 1;12(1):78-83
Petrosino S, Di Marzo V. The pharmacology of palmitoylethanolamide and first data on the therapeutic efficacy of some of its new formulations. Br J Pharmacol. 2017 Jun;174(11):1349-1365
Clayton P et al. Palmitoylethanolamide: A Natural Compound for Health Management. Int J Mol Sci. 2021 May 18;22(10):5305
Rankin L, Fowler CJ. The Basal Pharmacology of Palmitoylethanolamide. Int J Mol Sci. 2020 Oct 26;21(21):7942
Gatti A et al. Palmitoylethanolamide in the treatment of chronic pain caused by different etiopathogenesis. Pain Med. 2012 Sep;13(9):1121-30
Hanlon EC. Impact of circadian rhythmicity and sleep restriction on circulating endocannabinoid (eCB) N-arachidonoylethanolamine (anandamide). Psychoneuroendocrinology. 2020 Jan;111:104471
Pham L et al. The interplay between mast cells, pineal gland, and circadian rhythm: Links between histamine, melatonin, and inflammatory mediators. J Pineal Res. 2021 Mar;70(2): e 12699
Chaudhry SR et al. Melatonin Moderates the Triangle of Chronic Pain, Sleep Architecture and Immunometabolic Traffic. Biomedicines. 2021 Aug 9;9(8):984
Xie S et al. Role of Melatonin in the Regulation of Pain. J Pain Res. 2020 Feb 7;13:331-343
Maldonado MD et al. Evidence of melatonin synthesis and release by mast cells. Possible modulatory role on inflammation. Pharmacol Res. 2010 Sep;62(3):282-7
Dollins AB et al. Effect of inducing nocturnal serum melatonin concentrations in daytime on sleep, mood, body temperature, and performance. Proc Natl Acad Sci USA. 1994 Mar 1;91(5):1824-8
Chatterjea D et al. Mast cell degranulation mediates compound 48/80-induced hyperalgesia in mice. Biochem Biophys Res Commun. 2012 Aug 24;425(2):237-43

## Claims

1. Composition comprising palmitoylethanolamide (PEA) and melatonin, in formulation for oral, sublingual, rectal, transdermal or topical use comprising from 50 to 2400 mg of PEA and from 0.01 to 10 mg of melatonin, per single dose, in a weight ratio of PEA/melatonin from 100:1 to 120000:1.

2. Composition according to claim 1, comprising from 200 to 1200 mg of PEA and from 0.1 to 5 mg of melatonin, per single dose, in a weight ratio of PEA/melatonin from 1000:1 to 50000:1, preferably from 2000:1 to 15000:1, and more preferably from 4000:1 to 8000:1.

3. Composition according to claim 1 or 2, for use in the causal and not simply symptomatic treatment of pathologies sustained by, or attributable to, mast cell hyper-reactivity.

4. Composition according to claim 1 or 2, for use in the treatment or as an adjuvant in the treatment of pain and/or inflammation in a human subject.

5. Composition according to claim 1 or 2, for use according to claim 3 or 4, in formulation for oral or sublingual use comprising PEA and melatonin in a weight ratio of PEA/melatonin from 100:1 to 120000:1, preferably from 1000:1 to 50000:1, more preferably from 2000:1 to 15000:1 and even more preferably from 4000:1 to 8000:1.

6. Composition according to claim 1 or 2 for use according to claim 3 or 4, in formulation for sublingual use, comprising PEA and melatonin in a weight ratio of PEA/melatonin from 100:1 to 25000:1, preferably from 2000:1 to 10000:1, more preferably from 4000:1 to 8000:1.

7. Composition according to claim 1 or 2 for use according to claim 3 or 4, in rectal, transdermal or topical administration, comprising PEA and melatonin in a weight ratio of PEA/melatonin from 100:1 to 10000:1, preferably from 1000:1 to 8000:1, more preferably from 4000:1 to 8000:1.

8. Composition according to claim 1 or 2 for use according to claim 3 or 4, in cases of post-operative pain, peripheral neuropathies such as radiculopathy, carpal tunnel syndrome, diabetic neuropathy and post-herpetic neuralgia, migraine, fibromyalgia, osteoarthritis, cancer pain, psoriasis, allergic inflammation, and various types of chronic pelvic pain.

9. Composition for use according to claim 8, wherein pelvic pain is generated by a pathology selected from the group comprising Crohn's disease, irritable bowel syndrome, endometriosis, chronic prostatitis and vulvodynia.

10. Composition according to claim 1 or 2 for use according to any one of claims 3 to 9, in administration at any time of the day (morning, afternoon or evening).

11. Composition according to claim 1 or 2 for use according to any one of claims 3 to 10, further comprising a pharmaceutically or nutraceutically acceptable vehicle or excipient, or food vehicle or excipient.

12. Composition according to claim 1 or 2 for use according to any of claims 3 to 11, further comprising excipients selected from phospholipids, polyglycerol esters of fatty acids, mono- and diglycerides of fatty acids, medium-chain triglycerides, fractionated coconut oil, polyethoxylated castor oil, lime oil, olive oil, oat oil, sunflower lecithin, vitamin E acetate, silicon dioxide, microcrystalline cellulose, croscarmellose sodium, polyvinylpyrrolidone, magnesium stearate, sorbitol powder, sucrose, sucrose palmitate, polysorbate 80, anhydrous colloidal silica, corn starch, hydroxypropyl cellulose, talc, shellac, and mixtures thereof.

13. Composition according to claim 1 or 2 for use according to any one of claims 3 to 12, in which the PEA is micronized with a particle size of less than 10 µm or ultramicronized with a particle size of less than 5 µm.

14. Composition according to claim 1 or 2 for use according to any one of claims 3 to 13, in which melatonin is micronized with a particle size of less than 10 µm or ultramicronized with a particle size of less than 5 µm.
